(19) Europäisches Patentamt
European Patent Office
Office européen des brevets

(11) **EP 4 813 990 A1**

(12) **EUROPEAN PATENT APPLICATION**
published in accordance with Art. 153(4) EPC

(43) Date of publication:
**30.09.2026 Bulletin 2026/40**

(21) Application number: **24893274.1**

(22) Date of filing: **11.11.2024**

(51) International Patent Classification (IPC):
***C07D 487/04*** *(2006.01)* ***A61K 31/505*** *(2006.01)*
***A61K 31/519*** *(2006.01)* ***A61P 35/00*** *(2006.01)*

(52) Cooperative Patent Classification (CPC):
**A61K 31/505; A61K 31/519; A61K 39/395; A61P 35/00; C07D 487/04**

(86) International application number:
**PCT/CN2024/131172**

(87) International publication number:
**WO 2025/108116 (30.05.2025 Gazette 2025/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.11.2023 CN 202311568998**

(71) Applicant: **INNOLAKE BIOPHARMA (HANGZHOU) CO., LTD.**
**Hangzhou, Zhejiang 310018 (CN)**

(72) Inventors:
- **XIA, Mingde**
  **Hangzhou, Zhejiang 310018 (CN)**
- **CHEN, Rulei**
  **Hangzhou, Zhejiang 310018 (CN)**
- **ZHANG, Qian**
  **Hangzhou, Zhejiang 310018 (CN)**
- **LI, Yan**
  **Hangzhou, Zhejiang 310018 (CN)**

(74) Representative: **Epping - Hermann - Fischer Patentanwaltsgesellschaft mbH**
**Schloßschmidstraße 5**
**80639 München (DE)**

(54) **COMPOUND FOR TREATING CANCER**

(57) The present invention relates to compounds for treating cancer. In particular, the present invention relates to the use of the following compound or a pharmaceutically acceptable salt thereof in the preparation of a medicament for treating cancer:

wherein $R_1$ is ($C_1$-$C_6$) alkyl optionally substituted with hydroxyl; $R_2$ is independently halogen, ($C_1$-$C_6$) alkyl or halogen-substituted ($C_1$-$C_6$) alkyl; $R_3$ is independently ($C_1$-$C_6$) alkyl; m is 0, 1, 2, 3 or 4; n is 0, 1, 2, 3, 4 or 5. The compound can be used for treating cancer, especially solid tumors, and can inhibit tumor growth.

EP 4 813 990 A1

## Description

### Technical Field

The present invention relates to the field of pharmaceutical compounds, in particular to compounds for treating cancer.

### Background Art

Adenosine receptors are a class of purinergic G protein-coupled receptors, including four subtypes: A1, A2a, A2b and A3. The adenosine signaling pathway may serve as an important compensatory immunosuppressive mechanism. In solid tumors, hypoxic environment and decomposition of cellular tissues can produce a large amount of adenosine triphosphate (ATP).

CN110446712B discloses triazolopyrimidine derivatives as $A_{2A}$ receptor inhibitors, which can be used for treating diseases associated with $A_{2A}$ receptors, and this document is incorporated herein by reference.

CN112105617B discloses crystal forms of compounds.

There remains a demand for compounds for treating cancer, especially solid tumors.

### Summary of the Invention

An objective of the present invention is to provide compounds for treating cancer, especially solid tumors.

The present invention relates to the use of the following compound or a pharmaceutically acceptable salt thereof in the preparation of a medicament, wherein the medicament is used for treating cancer:

wherein:

$R_1$ is ($C_1$-$C_6$) alkyl optionally substituted with hydroxyl;
$R_2$ is independently halogen, ($C_1$-$C_6$) alkyl or halogen-substituted ($C_1$-$C_6$) alkyl;
$R_3$ is independently ($C_1$-$C_6$) alkyl;
m is 0, 1, 2, 3 or 4;
n is 0, 1, 2, 3, 4 or 5.

The compound can be used for treating cancer, especially solid tumors, and can inhibit tumor growth.

### Brief Description of the Drawings

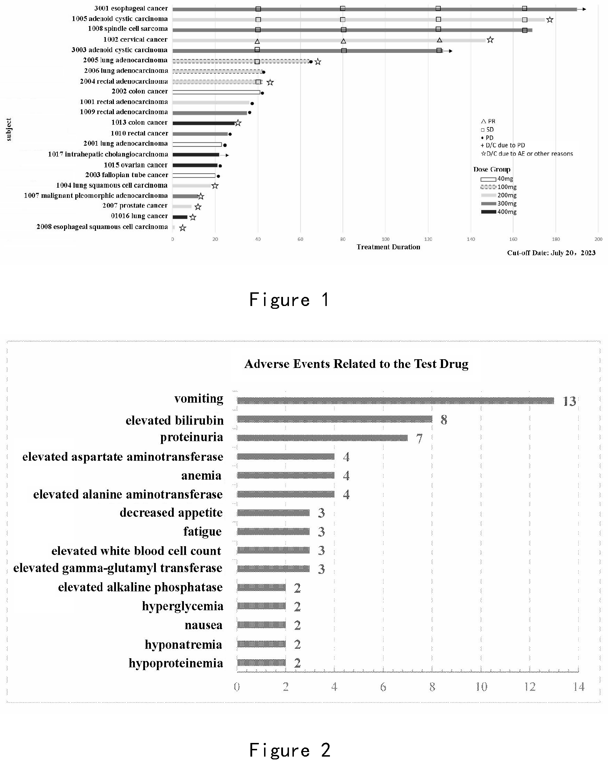

FIG. 1 is a diagram showing the treatment days of subjects;

FIG. 2 is a diagram showing adverse events related to the test drug;

FIG. 3 is a diagram showing the occurrence of adverse events related to the test drug in each dose group; and

FIG. 4 is an X-ray powder diffraction pattern of Crystal Form A of the compound.

### Detailed Description of the Embodiments

Unless otherwise defined, all technical and scientific terms used herein have the same meaning as commonly

understood by those skilled in the art. In case of conflict, the present document including definitions shall prevail. Preferred methods and materials are described below, but methods and materials similar or equivalent to those described herein can be used in the practice or testing of the present invention. The materials, methods and examples disclosed herein are illustrative only and not intended to be limiting.

The present invention provides the use of the following compound or a pharmaceutically acceptable salt thereof in the preparation of a medicament, wherein the medicament is used for treating cancer:

Wherein:

$R_1$ is ($C_1$-$C_6$) alkyl optionally substituted with hydroxyl;
$R_2$ is independently halogen, ($C_1$-$C_6$) alkyl or halogen-substituted ($C_1$-$C_6$) alkyl;
$R_3$ is independently ($C_1$-$C_6$) alkyl;
m is 0, 1, 2, 3 or 4;
n is 0, 1, 2, 3, 4 or 5.

In one embodiment, $R_1$ is hydroxyl-substituted ($C_1$-$C_6$) alkyl, preferably hydroxyl-substituted ($C_1$-$C_3$) alkyl, more preferably hydroxymethyl.
In one embodiment, $R_2$ is independently trifluoromethyl or ($C_1$-$C_6$) alkyl, and the ($C_1$-$C_6$) alkyl is preferably methyl.
In one embodiment, m is 2.
In one embodiment, n is 0.
In one embodiment, the compound is

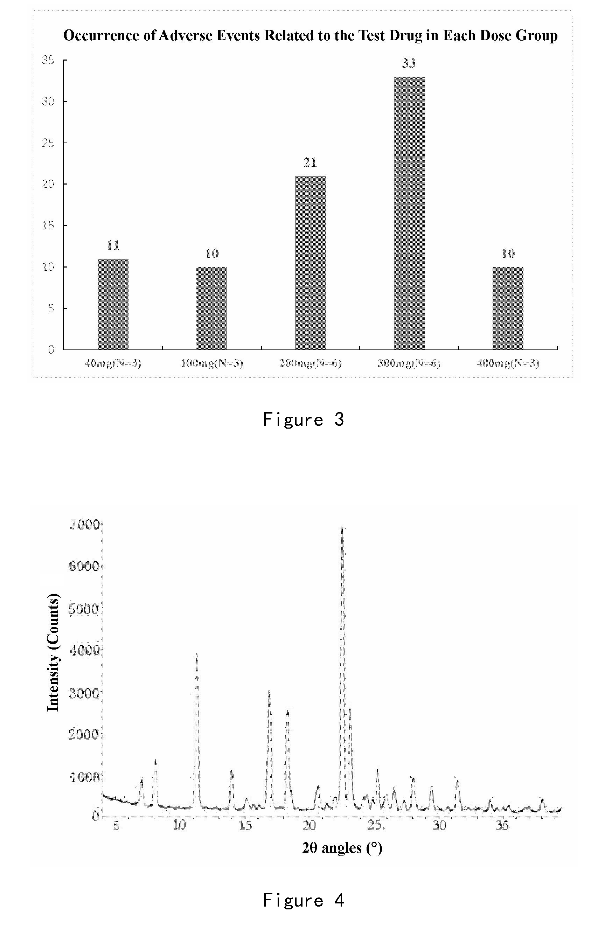

(designated as ILB-2109).
Compound ILB-2109 can be prepared according to the methods described in CN110446712B or CN112105617B, both of which are incorporated herein by reference.
In one embodiment, the cancer is a solid tumor.
In one embodiment, the cancer is selected from gastric cancer, esophageal cancer, urothelial carcinoma, prostate cancer, renal cancer, colorectal cancer, non-small cell lung cancer, triple-negative breast cancer, liver cancer, pancreatic cancer, head and neck squamous cell carcinoma, melanoma, cervical cancer, ovarian cancer, endometrial cancer, sarcoma, glioma, rectal cancer, lung cancer, adenoid cystic carcinoma, spindle cell sarcoma, lung adenocarcinoma, colon cancer, fallopian tube cancer, esophageal squamous cell carcinoma, rectal adenocarcinoma, lung squamous cell carcinoma, malignant pleomorphic adenocarcinoma, intrahepatic cholangiocarcinoma or nasopharyngeal carcinoma.
In one embodiment, the compound has Crystal Form A, whose X-ray powder diffraction pattern has characteristic diffraction peaks at the following 2θ angles: 8.08±0.2°, 11.30±0.2°, 14.00±0.2°, 16.90±0.2°, 18.30±0.2°, 22.52±0.2°, 23.15±0.2°, 25.26±0.2°.
In one embodiment, the compound has Crystal Form A, and its X-ray powder diffraction pattern is as shown in FIG. 4.
Crystal Form A of the compound can be prepared according to the method described in CN112105617B, which is

incorporated herein by reference.

In one embodiment, the compound is a deuterated compound.

The compound can form a deuterated compound by replacing hydrogen with deuterium. The bond formed by deuterium and carbon is stronger than that formed by ordinary hydrogen and carbon. Compared with non-deuterated compounds, deuterated compounds have the advantages of reducing toxic and side effects, increasing compound stability, enhancing therapeutic effect and prolonging compound half-life.

In one embodiment, the deuterated compound is selected from the group consisting of:

In one embodiment, the medicament further comprises an anti-CTLA-4 monoclonal antibody. Preferably, the weight ratio of the compound to the anti-CTLA-4 monoclonal antibody is 5:1 to 50:1, such as 10:1 to 20:1. Preferably, the medicament is used for treating colon cancer. The compound and the anti-CTLA-4 monoclonal antibody have a synergistic effect in treating cancer, such as solid tumors including colon cancer.

The compound can be formulated into conventional preparations in the art together with conventional auxiliary materials, carriers or excipients for administration to individuals. The preparations include tablets, capsules, injections, powders, granules, inhalants, suppositories, solutions, emulsions, suspensions, ointments, membrane preparations and the like.

**Example 1. Preclinical Research**

**Preclinical Pharmacodynamic Study**

In vitro activity detection results showed that ILB-2109 can efficiently inhibit the binding of A2aR to its isotopically labeled ligand with an IC50 of 2.92 nM. Meanwhile, ILB-2109 had high selectivity for A2aR (compared with A1, A2b and A3 receptors, the selectivity was 18.9 times, 40.7 times and 3424.7 times respectively). At the cellular level, ILB-2109 can also efficiently antagonize the activity of A2aR with an IC50 of 0.43 nM; under tumor physiological equivalent conditions (NECA 5 $\mu$M), the IC50 was 1.08 nM, also showing efficient antagonistic activity against A2aR. The antagonistic activity and selectivity of ILB-2109 against A2aR were comparable to those of the reference compound AZD4635 in Phase II clinical research.

In the in vivo pharmacodynamic evaluation of mouse colon cancer MC38 tumor model, ILB-2109 (50 and 100 mg/kg) combined with anti-CTLA-4 monoclonal antibody (5 mg/kg) also showed significant tumor inhibitory effects (TGI were 70.97% and 84.38% respectively).

**Experimental Method**

A total of 6 groups were set up in the experiment: vehicle control group, ILB-2109 low-dose and high-dose single-agent groups, Anti-CTLA-4 antibody single-agent group, and two dose groups of ILB-2109 and Anti-CTLA-4 in combination. The dosage and grouping are shown in Table 1.

**Table 1. Experimental Administration Dosage and Grouping**

| Group | Test Sample | Number of Animals | Administration Route | Single Administration Dosage | Administration Frequency** |
|---|---|---|---|---|---|
| 1 | Vehicle 1 + Vehicle 2* | 8 | Gavage + Intraperitoneal Injection | N/A + N/A | Twice a day + Twice a week |
| 2 | ILB-2109 + Vehicle 2 | 8 | Gavage + Intraperitoneal Injection | 50 mg/kg + N/A | Twice a day + Twice a week |
| 3 | Vehicle 1 + Anti-CTLA-4 | 8 | Gavage + Intraperitoneal Injection | N/A + 5 mg/kg | Twice a day + Twice a week |
| 4 | ILB-2109 + Anti-CTLA-4 | 8 | Gavage + Intraperitoneal Injection | 50 mg/kg + 5 mg/kg | Twice a day + Twice a week |

(continued)

| Group | Test Sample | Number of Animals | Administration Route | Single Administration Dosage | Administration Frequency** |
|---|---|---|---|---|---|
| 5 | ILB-2109 + Vehicle 2 | 8 | Gavage + Intraperitoneal Injection | 100 mg/kg + N/A | Twice a day + Twice a week |
| 6 | ILB-2109 + Anti-CTLA-4 | 8 | Gavage + Intraperitoneal Injection | 100 mg/kg + 5 mg/kg | Twice a day + Twice a week |
| Notes: a: The vehicle control group was Vehicle 1 + Vehicle 2 (Vehicle 1: 1% Tween80 + 9% PEG400 + 90% ddwater, Vehicle 2: DPBS). b: Vehicle 1 and ILB-2109 were administered starting on the day of inoculation, with an interval of 6 hours between twice daily administrations; Vehicle 2 and Anti-CTLA-4 antibody were administered on Days D9, D13, D16 and D20 after inoculation. | | | | | |

**Compound Preparation**

Anti-CTLA-4 antibody was aseptically diluted to 0.5 mg/mL with DPBS and prepared immediately before administration.

ILB-2109 was formulated to 10 mg/mL with vehicle, 1% tween80 + 9% PEG400 + 90% ddwater. Specific preparation method: Prepare Vehicle 1 (10 mL Tween80, 90 mL PEG400, 900 mL ddwater), weigh 1120 mg of ILB-2109 and add into 112 mL of Vehicle 1, grind to basically uniform, then ultrasonicate for about 2-3 minutes to obtain a homogeneous suspension. An appropriate amount of the solution was diluted to 5 mg/mL with Vehicle 1 (1% Tween80 + 9% PEG400 + 90% ddwater) at a ratio of 1:1. The prepared solution was aliquoted into 5 mL EP tubes with 4 mL per tube and stored at 4°C. Prepared once a week.

**Construction of MC38 Tumor Xenograft Model**

48 female C57BL/6 mice were inoculated subaxillarily with 0.1 mL DPBS containing 2×10^5 MC38 cells. On the day of inoculation, mice were randomly divided into vehicle control group, ILB-2109 low-dose group and ILB-2109 high-dose group according to body weight, with 16 animals in each group. The day of inoculation was defined as D1. On D9 after inoculation, the average tumor volume of the vehicle control group reached about 64 mm^3. According to animal tumor volume and body weight, the original vehicle control group was divided into vehicle control group and Anti-CTLA-4 antibody single-agent group; the original ILB-2109 low-dose group was divided into ILB-2109 low-dose single-agent group and the combination group of low-dose ILB-2109 and anti-CTLA-4 antibody; the original ILB-2109 high-dose group was divided into ILB-2109 high-dose single-agent group and the combination group of high-dose ILB-2109 and Anti-CTLA-4 antibody. Administration was carried out for each group on the day of regrouping according to the set dosage.

Tumor volume was measured and body weight was weighed 3 times a week.

**Sample Collection**

All animals were euthanized on D23 after inoculation, and tumor samples were collected and weighed.

**Observation Indicators**

**Tumor Volume**

Tumor volume was measured 3 times a week, and tumor growth inhibition TGITV (%) was calculated. The calculation formula of Tumor Volume (TV) is:

$$TV = 1/2 \times a \times b^2$$

where a and b represent the major diameter and minor diameter of the tumor mass respectively.

Tumor growth inhibition TGITV (%) = [(1 - Mean tumor volume at the end of treatment group administration)/(Mean tumor volume of solvent control group at the end of treatment)] × 100%.

## Tumor Weight

Tumor weight of animals was measured during sample collection. The tumor growth inhibition was calculated according to the following formula:

$$\text{Tumor Growth Inhibition based on Tumor Weight TGITW (\%)} = (\text{TWc - TWt}) / \text{TWc} \times 100\%$$

where TWt is the mean tumor weight of the treatment group, and TWc is the mean tumor weight of the vehicle control group.

## Data Processing and Statistical Analysis

All data were expressed as Mean ± SEM.

In this experiment, data on D21 after inoculation were used for statistical analysis by PRISM software. T test was used for comparison between two groups, and one-way ANOVA was used for comparison among three or more groups.

## Experimental Results

## Tumor Volume

This experiment evaluated the efficacy of ILB-2109 alone and in combination with anti-CTLA-4 antibody (Clone 9D9) in MC38 mouse colon cancer xenograft model. On Day 21 of administration, the T/C of the combination group of high-dose ILB-2109 and Anti-CTLA-4 antibody was 15.62%, and TGI was 84.38%. (See Table 2 and Table 3 for details). Among them, the tumor volumes of all animals in the combination group of high-dose ILB-2109 and Anti-CTLA-4 antibody were less than 250 mm^3, only 5 mice in the antibody single-agent group had tumor volume less than 250 mm^3, and 1 animal in the vehicle group had tumor volume less than 250 mm^3.

**Table 2. Tumor Inhibitory Effect of ILB-2109 on MC38 Mouse Xenograft Model**

| Group | Tumor Volume ($mm^3$)[a] (Day 9) | Tumor Volume ($mm^3$)[a] (Day 12) | Tumor Volume ($mm^3$)[a] (Day 15) | TGI (%) (Day 21)[b] | T/C (%) (Day 21)[b] | Number of Animals with Tumor Volume > $250mm^3$ |
|---|---|---|---|---|---|---|
| Vehicle Control (n=8) | 69±7 | 700±103 | 276±34 | N/A | N/A | 7/8 |
| ILB-2109 (50 mg/kg, n=8) | 63±6 | 494±87 | 225±34 | 29.39 | 70.61 | 8/8 |
| Anti-CTLA-4 Antibody (5 mg/kg, n=8) | 66±3 | 181±39 | 171±25 | 74.13 | 25.87 | 3/8 |
| ILB-2109+CTLA-4 (50+5mg/kg, n=8) | 63±6 | 203±30 | 141±11 | 70.97 | 29.03 | 2/8 |
| ILB-2109 (100mg/kg, n=8) | 58±4 | 369±54 | 257±18 | 47.24 | 52.76 | 5/8 |
| ILB-2109+CTLA-4 (100+5mg/kg, n=8) | 58±5 | 109±21 | 90±11 | 84.38 | 15.62 | 0/8 |
| Notes: a. Mean ± SEM.<br>b. Tumor growth inhibition was calculated by T/C and TGI (TGI (%) = [1-(T20)/(V20)] ×100). | | | | | | |

Table 3. P Values of Tumor Volume (TV) Comparison Among Groups in MC38 Mouse Xenograft **Model[a]**

| Group | G1 | G2 | G3 | G4 | G5 | G6 |
|---|---|---|---|---|---|---|
| Vehicle Control(n=8) | N/A | 0.2173 | <0.0001 | <0.0001 | 0.0078 | <0.0001 |
| ILB-2109(50 mg/kg,n=8) | 0.2173 | N/A | 0.0133 | 0.0257 | 0.7291 | 0.0013 |
| Anti-CTLA-4 Antibody(5 mg/kg,n=8) | <0.0001 | 0.0133 | N/A | 0.9999 | 0.3055 | 0.9656 |
| ILB-2109+CTLA-4(50+5mg/kg,n=8) | <0.0001 | 0.0257 | 0.9999 | N/A | 0.4426 | 0.8982 |
| ILB-2109(100mg/kg,n=8) | 0.0078 | 0.7291 | 0.3055 | 0.4426 | N/A | 0.0605 |

(continued)

| Group | G1 | G2 | G3 | G4 | G5 | G6 |
|---|---|---|---|---|---|---|
| ILB-2109+CTLA-4(100+5mg/kg,n=8) | <0.0001 | 0.0013 | 0.9656 | 0.4426 | 0.8982 | N/A |
| Notes: P values were obtained by one-way ANOVA analysis of tumor volume, and Tukey's method was used for inter-group comparison.<br>a. Analytical values were tumor volume data of each group on Day 21 of administration.<br>G1: Vehicle Control (n=8)<br>G2: ILB-2109-50mg/kg (n=8)<br>G3: Anti-CTLA-4 Antibody-5mg/kg (n=8)<br>G4: ILB-2109+Anti-CTLA-4-(50+5)mg/kg (n=8)<br>G5: ILB-2109-100mg/kg (n=8)<br>G6: ILB-2109+Anti-CTLA-4-(100+5)mg/kg (n=8) | | | | | | |

**Table 4. Tumor Volume of Each Group at Different Time** Points

| Days After Inoculation | Tumor Volume ($mm^3$)[a] | | | | | |
|---|---|---|---|---|---|---|
| | G1 | G2 | G3 | G4 | G5 | G6 |
| 9 | 69±7 | 63.65±6 | 66.50±3 | 63±6 | 58±4 | 58±5 |
| 11 | 127±14 | 115±13 | 102±9 | 105±8 | 78±8 | 75±5 |
| 13 | 194±22 | 143±20 | 122±15 | 114±11 | 105±11 | 76±5 |
| 15 | 276±34 | 225±34 | 171±25 | 141±11 | 157±18 | 90±10 |
| 17 | 393±52 | 269±41 | 189±28 | 152±21 | 207±25 | 92±12 |
| 21 | 700±103 | 494±87 | 181±35 | 203±30 | 369±54 | 109±21 |

Tumor Weight

Tumor tissues were collected at the end of the experiment, weighed, and Tumor Growth Inhibition based on Tumor Weight (TGITW) was calculated based on tumor weight data. The results showed that the TGITW of ILB-2109 low-dose single-agent group was 31%; the TGITW of Anti-CTLA-4 antibody group was 83%, which showed significant tumor inhibitory effect compared with the vehicle control group (p=0.0001); the TGITW of the combination group of low-dose ILB-2109 and anti-CTLA-4 antibody reached 74%, which showed significant tumor inhibitory effect compared with the vehicle control group (p=0.0001), and the efficacy was better than that of any single agent.

The TGITW of ILB-2109 high-dose single-agent group reached 38%. The TGITW of the combination group of high-dose ILB-2109 and Anti-CTLA-4 antibody reached 89%, which showed significant tumor inhibitory effect compared with the vehicle control group (p=0.0001). The result was better than those of the low-dose combination group and the antibody single-agent group. See Table 5 for details.

**Table 5. Tumor Inhibitory Effect of Test Substances on MC38 Cell Xenograft Model in Mice**

| Group | Tumor Weight (g)[a] | $TGI_{TW}$ (%) | P Value[b] |
|---|---|---|---|
| Vehicle Control | 1.0155±0.1276 | - | - |
| ILB-2109(50 mg/kg) | 1.7030±0.1419 | 31 | 0.1563 |
| Anti-CTLA-4 Antibody(5 mg/kg) | 0.1718±0.0415 | 83 | 0.0001 |
| ILB-2109+Anti-CTLA-4 Antibody (50+5)mg/kg | 0.2653±0.0502 | 74 | 0.0001 |
| ILB-2109(100 mg/kg) | 0.6318±0.1355 | 38 | 0.0461 |

(continued)

| Group | Tumor Weight (g)[a] | $TGI_{TW}$ (%) | P Value[b] |
|---|---|---|---|
| ILB-2109+Anti-CTLA-4 Antibody (100+5)mg/kg | 0.1163±0.0210 | 89 | 0.0001 |
| Notes: a. Mean ± SEM.<br>b. P values were obtained by ONE-WAY ANOVA analysis of tumor weight. Anti-CTLA-4 antibody single-agent group vs low-dose combination group, p=0.7595; Anti-CTLA-4 antibody single-agent group vs high-dose combination group, p=0.9090; ILB-2109 low-dose single-agent group vs low-dose combination group, p=0.0100; ILB-2109 high-dose single-agent group vs high-dose combination group, p=0.0007. | | | |

**Experimental Conclusion**

In this MC38 mouse colon cancer xenograft model experiment, ILB-2109 combined with Anti-CTLA-4 antibody at doses of 50 mg/kg and 100 mg/kg showed anti-tumor efficacy.

Compared with the Anti-CTLA-4 antibody single-agent group, the tumor volume of the combination group of 100 mg/kg ILB-2109 and Anti-CTLA-4 antibody showed a decreasing trend without statistical difference. Further analysis showed that the proportion of mice with tumor volume >250 mm^3 in the combination group (0/8) was significantly lower than that in the Anti-CTLA-4 antibody single-agent group (3/8).

**Example 2 Preclinical Pharmacokinetic Study**

According to the Technical Guidelines for Non-clinical Pharmacokinetic Studies of Drugs and the results of in vitro metabolic species difference studies, liquid chromatography-tandem mass spectrometry (LC-MS/MS) was used in this pharmacokinetic study to fully verify the analytical method for the determination of ILB-2109 content in plasma (EDTA-K2 anticoagulation) of Sprague-Dawley rats and cynomolgus monkeys. The linear range of the method was 20.0~20000 nM. The precision, accuracy and other indicators of the method were confirmed to comply with the relevant regulations of biological sample detection.

**Absorption**

According to existing classification standards, ILB-2109 exhibited high permeability in Caco-2 cells, and the test substance was most likely a substrate of efflux transporters.

**SD Rat Absorption Test**

This experiment studied the pharmacokinetic properties of ILB-2109 in female and male SD rats, including 1) 2 mg/kg single intravenous injection administration study; 2) 12, 40 and 120 mg/kg oral dose escalation study; 3) 40 mg/kg repeated oral administration BID for 7 consecutive days.

After single intravenous injection of 2 mg/kg ILB-2109 to female and male SD rats respectively, the plasma clearance rate (CL) was 1.33±0.135 mL/min/kg and 6.30±1.04 mL/min/kg respectively, the steady-state apparent volume of distribution (Vdss) was 0.526±0.0558 L/kg and 0.681±0.0975 L/kg respectively, the elimination half-life (T1/2) and the area under the plasma concentration-time curve from time zero to the last quantifiable time point (AUC0-last) were 5.71±1.25 h and 60300±5180 nM•h, 1.26±0.361 h and 13300±2260 nM•h respectively.

After single oral administration of 12, 40 and 120 mg/kg ILB-2109 to male SD rats, AUC0-last were 58900 ±19200, 296000±54100 and 792000±167000 nM•h respectively, peak concentrations (Cmax) were 23800±2230, 63300±1650 and 118000±19800 nM respectively, and peak time appeared at 0.667±0.289, 1.33±0.577 and 2.67±1.15 h after administration respectively. The bioavailability was 73.0% when ILB-2109 was orally administered at 12 mg/kg.

After single oral administration of 12, 40 and 120 mg/kg ILB-2109 to female SD rats, AUC0-last were 247000 ±22100, 915000±169000 and 1560000±205000 nM•h respectively, peak concentrations (Cmax) were 33700±2800, 124000±17500 and 155000±24300 nM respectively, and peak time appeared at 2.33±1.53, 3.33±1.15 and 2.67±1.15 h after administration respectively. The bioavailability was 67.8% when ILB-2109 was orally administered at 12 mg/kg.

At each oral single dose of 12, 40 and 120 mg/kg, the ratios of AUC0-last between female and male rats were 4.19, 3.09 and 1.97 respectively, indicating significant differences in systemic exposure between female and male rats at low and medium doses. Within the dose range of 12 to 40 mg/kg, the systemic exposure (AUC0-last) and Cmax of female and male rats increased basically proportionally to the dose. After continuous oral administration of 40 mg/kg ILB-2109 for 7 days, the ratios of Cmax on Day 7 to Day 1 in female and male rats were 0.899 and 0.673 respectively, and the ratios of AUC0-last on Day 7 to Day 1 in female and male rats were 0.669 and 0.584 respectively.

All animals tolerated the compound well without any abnormal manifestations.

**Cynomolgus Monkey Absorption Test**

This experiment studied the pharmacokinetic properties of ILB-2109 in female and male cynomolgus monkeys, including 1) 3 mg/kg single intravenous injection administration study; 2) 3, 15 and 60 mg/kg oral dose escalation study; 3) 15 mg/kg oral administration BID for 7 consecutive days.

After single intravenous injection of 3 mg/kg to female and male cynomolgus monkeys, the plasma clearance rate (CL) of ILB-2109 was 3.13±0.361 mL/min/kg, the steady-state apparent volume of distribution (Vd) was 0.867±0.141 L/kg, the elimination half-life (T1/2) and the area under the plasma concentration-time curve from time zero to the last quantifiable time point (AUC0-last) were 4.33±0.750 h and 39500±5270 nM•h respectively.

After single oral administration of 3, 15 or 60 mg/kg ILB-2109 to female and male cynomolgus monkeys, peak concentration (Cmax) were 8190±1810, 33900±9660 and 59300±33300 nM respectively, and peak time appeared at 1.33±0.516, 2.00±1.10 and 4.00±2.19 h after administration respectively. AUC0-last were 35200±6870, 192000 ±51600 and 571000±254000 nM•h respectively. The bioavailability of the 3 mg/kg oral dose group was 89.1%.

After oral administration of 15 mg/kg ILB-2109 twice a day for 7 consecutive days to female and male cynomolgus monkeys, the peak concentration (Cmax) on Day 1 and Day 7 were 18200±4000 and 28300±4780 nM respectively, and peak time appeared at 3.00±1.10 h after administration. AUC0-last were 172000±34400 and 268000±42500 nM•h respectively.

With the oral dose increasing from 3 to 60 mg/kg, the systemic exposure (AUC0-last) of ILB-2109 in female and male cynomolgus monkeys increased basically proportionally to the dose.

There was no significant gender difference in systemic exposure (AUC0-last and Cmax) of female and male cynomolgus monkeys at each oral dose.

After oral administration of 15 mg/kg ILB-2109 twice a day for 7 consecutive days, there was no obvious accumulation of systemic exposure in female and male cynomolgus monkeys.

**Distribution**

ILB-2109 showed a high binding rate (95.1-95.8%) in human plasma, and a moderate binding rate (87.3%-95.0%) in plasma of CD-1 mice, Sprague-Dawley rats, Beagle dogs and cynomolgus monkeys, without obvious concentration dependence.

After single oral administration of 30 mg/100 μCi/kg [14C] ILB-2109 to female and male rats, total radioactivity was widely distributed in rats, mainly distributed in liver, kidney, lung and gastrointestinal wall, and a certain degree of distribution was detected in all tested tissues including mesenteric lymph nodes, submandibular lymph nodes, axillary lymph nodes and thymus. The Tmax in most tissues and plasma of female rats was 4 hours, while that of male rats was 0.25 hours, indicating that the absorption of total radioactivity in male rats was slightly faster than that in female rats. After single oral administration of 30 mg/100 μCi/kg [14C] ILB-2109 to female and male rats, total radioactivity was eliminated rapidly in vivo. At 4 hours after administration, it decreased to a certain extent in most tissues and plasma of male rats, indicating that male rats entered the elimination stage earlier. At 24 hours after administration, radioactivity distribution could still be detected in most tissues and plasma of female rats, while it was below the lower limit of detection in most tissues and plasma of male rats. At the last collection time point (72 hours), only a small amount of radioactivity distribution remained in liver, intestinal wall and plasma of female rats among all tissues of female and male rats, accounting for about 0.09% of the administered dose in total, and total radioactivity in other tested tissues and plasma was below the lower limit of detection.

**Metabolism**

ILB-2109 was metabolized at a moderate rate in CD-1 mice and SD rats, and slowly metabolized or non-metabolized in Beagle dogs, cynomolgus monkeys and human liver microsomes. The main biotransformation pathways of ILB-2109 in in vitro liver microsomes and hepatocytes include oxidation, dehydrogenation and glucuronidation (hepatocyte biotransformation pathway), and no human-specific metabolites were detected.

In vivo metabolism experiments in SD rats showed that the main clearance pathways of [14C]ILB-2109 in rats were direct glucuronidation to form M576a/b, or mono-oxidation followed by glucuronidation to form M592a/b/c, mainly excreted via feces after liver metabolism, and a small amount excreted via kidney. Glucuronide conjugates in feces could be partially converted back to the mono-oxidized product M416a/b and parent drug form; the secondary clearance pathway was mono-oxidation and dehydrogenation to form M414, excreted from liver and kidney.

In in vivo metabolism experiments of cynomolgus monkeys, the relative abundance of parent drug ILB-2109 in plasma samples of male and female monkeys was more than 83%, which was the main component in plasma samples. ILB-2109 was mainly metabolized via oxidation, dehydrogenation and glucuronidation in monkey plasma. For the

production of ILB-2109 metabolite M4, CYP3A was the main metabolic enzyme, CYP2B6, CYP2C8 and CYP2C9 may play a secondary role in the production of M4, and other isoenzymes (CYP1A2, CYP2C19 and CYP2D6) had little or no effect.

## Excretion

Within 0-168 hours after single oral administration to female and male intact rats, the average of total radioactivity recovery was 91.74%; among them, total urinary excretion accounted for 13.81% of the administered dose, feces accounted for 75.17%, cage washing and cleaning fluid accounted for 2.75%. Excretion mainly occurred within 48 hours after administration, accounting for about 88.01% of the administered dose.

Within 0-72 hours after single oral administration to female and male bile duct cannulated (BDC) rats, the average of total radioactivity recovery was 93.63%; among them, total biliary excretion accounted for 66.17% of the administered dose, urine accounted for 12.16%, feces accounted for 14.45%, cage washing and cleaning fluid accounted for 0.85%. According to the total radioactive excretion of bile and urine in BDC rats, the oral absorption rate of ILB-2109 was at least 78.33%.

## Drug-Drug Interaction

In vitro human liver microsome tests showed that ILB-2109 had weak inhibitory effect on CYP2C8 (IC50=33.9μM), no inhibitory effect on CYP2C9 (IC50=57.2μM) and CYP2C19 (IC50=91.6μM), and no inhibitory effect on CYP1A2, CYP2B6, CYP2D6, CYP3A4 (using midazolam as substrate) and CYP3A4 (using testosterone as substrate) (IC50 > 100 μM). ILB-2109 had no time-dependent inhibitory effect on CYP1A2, CYP2B6, CYP2C8, CYP2C9, CYP2C19, CYP2D6 and CYP3A4.

The non-dilution method was used to determine the time-dependent inhibitory effect of ILB-2109 on human liver microsomal cytochrome P450 isoenzymes (CYP1A2, CYP2B6, CYP2C8, CYP2C9, CYP2C19, CYP2D6 and CYP3A4). Generally, an IC50 shift multiple ≥1.5 was taken as the judgment standard for time-dependent inhibitors. The results showed that ILB-2109 had no time-dependent inhibitory effect on CYP1A2, CYP2B6, CYP2C8, CYP2C9, CYP2C19, CYP2D6 and CYP3A4.

In vitro hepatocyte induction assay was used to evaluate the effect of ILB-2109 on the enzyme activity and gene expression level of cytochrome P450 isoenzymes CYP1A2, CYP2B6 and CYP3A4. In vitro enzyme activity data showed that ILB-2109 was not an inducer of CYP1A2, CYP2B6 or CYP3A4 at a concentration of 0.200, 2.00 or 20.0 μM. In vitro gene expression level data showed that at ILB-2109 concentration of 2.00 μM, results of Donor 1 and Donor 3 indicated that ILB-2109 was an inducer of cytochrome P450 isoenzyme CYP3A4. At ILB-2109 concentration of 20.0 μM, results of Donor 1 indicated that ILB-2109 was an inducer of cytochrome P450 isoenzymes CYP1A2 and CYP2B6; results of Donor 1, 2 and 3 indicated that ILB-2109 was an inducer of hepatocyte cytochrome P450 isoenzyme CYP3A4.

## Example 3 Preclinical Toxicological Study

In accordance with the relevant requirements of the Good Laboratory Practice for Non-clinical Drug Studies issued by the National Medical Products Administration of the People's Republic of China, and following the test protocol and revisions, the safety pharmacology test, single-dose toxicity, genetic toxicity, etc. of ILB-2109 were explored.

## Single-Dose Toxicity Study

SD rats were given single intragastric administration of ILB-2109 and observed for 14 days. The single doses were 0 (vehicle), 500, 1000 and 2000 mg/kg ILB-2109. The results showed that all animals survived until scheduled dissection, no dying or death occurred, and no obvious clinical symptoms were observed. No abnormalities were found in body weight, food consumption, clinical examination and gross anatomy. Under the test conditions, the maximum tolerated dose (MTD) of ILB-2109 in rats was 2000 mg/kg.

Cynomolgus monkeys were given single intragastric administration of 0 (vehicle), 250, 500 and 1000 mg/kg ILB-2109 and observed continuously for 14 days after single administration. The results showed that all animals survived until scheduled dissection without dying or death. Within 30 min ~ 4 h after administration on the day of administration, mild vomiting (a small amount of food-like vomit) occurred in medium and high-dose groups. No abnormalities were found in body weight, food consumption, body temperature, respiration, electrocardiogram, blood routine, ophthalmic examination, clinical examination, bone marrow and gross anatomy. Under the test conditions, the maximum tolerated dose (MTD) of cynomolgus monkeys given single intragastric administration of ILB-2109 within 24 h was 1000 mg/kg. The test substance had effects on digestive system (gastrointestinal function, liver function).

**Repeated-Dose Toxicity Test**

SD rats were given continuous intragastric administration of ILB-2109 at doses of 0 (vehicle), 60, 200 and 600 mg/kg for 28 consecutive days. The results showed that no test substance-related clinical symptoms, changes in body weight and food consumption were observed in each dose group. WBC and #LYMPH increased or tended to increase in medium and high-dose groups; GLU decreased or tended to decrease in medium and high-dose groups; CREA and UREA increased or tended to increase in female high-dose group; GLOB increased and A/G decreased in female animals of each dose group. Histopathological examination only showed centrilobular hepatocyte hypertrophy in female animals of medium and high-dose groups. All the above changes completely recovered during the recovery period. There was significant gender difference in AUC0-24h in the low-dose group, and no significant gender difference in systemic exposure in other dose groups; no obvious accumulation of systemic exposure was observed in each dose group; the increase ratio of systemic exposure was lower than the increase ratio of dose. Under the test conditions, the NOAEL of ILB-2109 administered intragastrically to SD rats for 4-week repeated-dose toxicity test was 60 mg/kg (at this dose, female AUC0-24h=295.88 h*μg/mL, Cmax=22.16 μg/mL; male AUC0-24h=114.50 h*μg/mL, Cmax=15.86 μg/mL), and MTD was 600 mg/kg (at this dose, female AUC0-24h=724.86 h*μg/mL, Cmax=69.40 μg/mL; male AUC0-24h=481.00 h*μg/mL, Cmax=36.34 μg/mL). ILB-2109 may have toxic effects on digestive system (liver function), blood system (granulocyte series) and urinary system (renal function) of SD rats, which can be reversed at the end of the recovery period.

40 cynomolgus monkeys (half male and half female) were randomly divided into 4 groups with 5 animals per gender in each group, and given intragastric administration of 0 (vehicle), 20, 60 and 200 mg/kg/day ILB-2109 once a day for 28 consecutive days. The results showed that 1 male animal in the high-dose group was found dying 2 h after administration on D3, with decreased respiratory frequency, abnormal breathing sound, prone position, abnormal feces and vomiting symptoms before death. Gross observation showed diffuse dark red lung and white foam in trachea. Combined with gross observation and histopathological examination results, the cause of animal death was considered to be related to lung injury. No test substance-related clinical symptoms, changes in body weight and food consumption were observed in each dose group. %LYMPH decreased and %NEUT increased in male animals of medium-dose group; % LYMPH tended to decrease and %NEUT tended to increase in female and male animals of high-dose group. Histopathological examination only showed decreased adrenal cortical vacuoles in medium and high-dose groups. All the above changes completely recovered during the recovery period. In addition, no drug-related changes were found in body temperature, respiration, electrocardiogram, ophthalmic examination and bone marrow. There was no gender difference in systemic exposure in each dose group; no obvious accumulation; the increase ratio of systemic exposure was lower than the increase ratio of dose.

Therefore, under the test conditions, the HNSTD of cynomolgus monkeys given intragastric administration of ILB-2109 for 4-week repeated-dose toxicity test was 60 mg/kg (at this dose, female AUC0-24h=148.56 h*μg/mL, Cmax=11.92 μg/mL; male AUC0-24h=205.78 h*μg/mL, Cmax=14.32 μg/mL), and NOAEL was 20 mg/kg (at this dose, female AUC0-24h=60.97 h*μg/mL, Cmax=5.01 μg/mL; male AUC0-24h=80.19 h*μg/mL, Cmax=6.58 μg/mL). Attention should be paid to the potential impact of ILB-2109 on respiratory system, blood system, endocrine system and other systems in clinical use.

**Genetic** Toxicity Test

AMES

This test detected the ability of ILB-2109 to induce reverse mutation of histidine-deficient Salmonella typhimurium strains (TA97a, TA98, TA100, TA102 and TA1535) with and without exogenous metabolic activation system. The results showed that ILB-2109 at 128 ~ 5000 μg/plate had no mutagenicity to five strains of TA1535, TA102, TA100, TA98 and TA97a with or without S9 metabolic activation system. Therefore, under the research conditions, the Ames test result was negative.

Chromosome Aberration Test

The test observed the effect of ILB-2109 on chromosome structure and number of mammalian cells (CHL cells). The results showed that the chromosome aberration test result was negative when CHL cells were treated with ILB-2109 at 3.2, 16.0 and 80.0 μg/mL.

Micronucleus Test

SD rats were given intragastric administration of 500 ~ 2000 mg/kg ILB-2109. Each dose of ILB-2109 had no micronucleus-inducing effect on bone marrow polychromatic erythrocytes of SD rats.

Example 4. Multicenter, Open-label, Phase **Ia** Clinical Study: Dose Escalation and Dose Expansion Clinical Study of ILB-2109 Tablets in Patients with Advanced Solid Tumors

Test Drug: A2aR Antagonist ILB-2109

ILB-2109 tablets were prepared by granulation and tableting of ILB-2109 with conventional cellulose auxiliary materials.

**Research Stage: Phase Ia**

**Number of Subjects: A maximum of 78 subjects** are expected to be enrolled (27 ~ 54 in dose escalation stage, 12 ~ 24 in dose expansion stage).

**Research Cycle**

Screening Period (Day -28 to Day -1);
Single Administration Period (3 days);
Continuous Administration Period (Oral administration of ILB-2109 tablets under fasting condition, QD, continuous oral administration with 21 days as one cycle);
Safety Follow-up after Treatment (within 28 days after the last administration);
Survival Follow-up (follow-up every 3 months after safety follow-up until the end of the study).

**Termination of Treatment**

All patients will continue treatment until disease progression, intolerable toxicity, death, decision by the investigator or voluntary withdrawal of the subject.

**End of Study**

It is expected to end when the first administration of the last enrolled subject reaches 1 year or the trial is completed (whichever comes earlier). Completion of the trial refers to when the subject completes the last study-related telephone contact or visit, terminates the trial or loses follow-up (i.e., the investigator cannot contact the subject).

**Research Objectives**

**Primary Objectives**

To observe the safety and tolerability of single and continuous oral administration of ILB-2109 tablets in patients with advanced solid tumors;

To determine the maximum tolerated dose (MTD) of ILB-2109 tablets and the appropriate recommended dose (RD) for subsequent studies.

**Secondary Objectives**

To evaluate the pharmacokinetic characteristics of ILB-2109 tablets;

To preliminarily evaluate the effect of food on PK characteristics and safety tolerability of ILB-2109 tablets (expansion stage only);

To preliminarily evaluate the efficacy of ILB-2109 tablets in the treatment of patients with advanced solid tumors.

**Exploratory Objectives**

To describe the pharmacodynamic (PD) characteristics of ILB-2109 tablets, including signal levels of phosphorylated c-AMP response element-binding protein (pCREB), cytokines TNF-$\alpha$, IFN-$\gamma$ and IL-2 (dose escalation stage only);

To evaluate potential biomarkers of ILB-2109 tablets in peripheral blood and tumor specimens of patients with advanced solid tumors, which: (1) may be related to biological activity (pharmacodynamics); (2) may predetermine subjects most likely to respond to ILB-2109 tablet treatment (predictive).

**Research Design**

This is an open-label, non-randomized, multicenter clinical study including dose escalation and dose expansion stages of single-agent ILB-2109 tablets; a maximum of 78 subjects are expected to be enrolled.

**Dose Escalation Stage**

Patients with advanced solid tumors who have no standard effective treatment regimen, failed standard treatment or are not suitable for standard treatment at this stage are planned to be enrolled, and dose escalation adopts the traditional "3+3" rule.

ILB-2109 tablets have an administration plan of about 9 dose groups, as shown in Table 6 below. Subjects take medicine under fasting condition (at least 2 hours before or after meal), and no food is allowed within 1 hour after taking medicine. Each subject receives single administration on Day 1, followed by 3 days of PK blood sample collection and safety monitoring (Cycle 0). If no DLT occurs during the observation period, the patient will enter the continuous administration period (Cycle 1), start once daily administration with 21 days as one administration cycle until the subject has disease progression, intolerable toxicity, withdrawal of informed consent, death or other conditions requiring termination of treatment. The trial will determine the dose group entering the expansion stage according to the safety, preliminary efficacy and PK characteristics of ILB-2109 tablets.

**Table 6**

| Dose Group | Daily Total Dose mg | Administration Regimen | Daily Total Dose Increase % | Number of Subjects |
|---|---|---|---|---|
| 1 | 40 | 40mg QD | -- | 3 |
| 2 | 100 | 100mg QD | 150.0 | 3 |
| 3 | 200 | 200mg QD | 100.0 | 6 |
| 4 | 300 | 300mg QD | 50.0 | 6 |
| 5 | 400 | 400mg QD | 33.3 | 3~6 |
| 6 | 600 | 600mg QD | 50.0 | 3~6 |
| 7 | 800 | 800mg QD | 33.3 | 3~6 |
| 8 | 1000 | 1000mg QD | 25.0 | 3~6 |
| 9 | 1200 | 1200mg QD | 20.0 | 3~6 |

Note: Except Dose Groups 1-4 which have been completed, the 5th dose group is ongoing, and subsequent dose groups are tentative doses and frequency, which will be adjusted according to the safety, efficacy, pharmacokinetics and other data of previous dose groups. The total number of dose groups will also be increased or decreased according to actual conditions.

**Definition and Evaluation of DLT**

DLT is defined as follows: Any dose-limiting toxicity event occurring in the DLT observation period (total 24 days), including the single administration DLT observation period (3 days of single administration) and the continuous administration DLT observation period (21 days of the first cycle of continuous administration); DLT evaluation shall be conducted on C2D1; and all toxicities are graded according to NCI-CTCAE (Version 5.0). Any of the following adverse events related to the test drug occurring during the observation period shall be regarded as a DLT:

**Table 7**

| Toxicity | Any one of the following shall be regarded as DLT |
|---|---|
| Hematology | Grade 4 neutropenia lasting >3 days<br>≥Grade 3 febrile neutropenia |

(continued)

| | |
|---|---|
| | Grade 4 thrombocytopenia<br>Grade 3 thrombocytopenia accompanied by bleeding<br>Grade 4 anemia<br>Grade 5 hematological toxicity |
| Non-hematological Toxicity | ≥Grade 4 non-hematological toxicity<br>Grade 3 non-hematological toxicity that cannot recover to ≤Grade 2 within 3 days after treatment (excluding asymptomatic simple laboratory abnormalities judged by the investigator without intervention needed)<br>Grade 2 interstitial pneumonia |
| Other Adverse Events | Other toxic reactions judged by the investigator requiring permanent discontinuation of the study drug or resulting in less than 75% of the planned administration of the study drug during the DLT observation period |
| Note | Toxicity events and severity grading are defined by CTCAE Version 5.0;<br>Supportive treatment shall be given according to local routine treatment. |

**Tumor Evaluation**

Tumor evaluation adopts Response Evaluation Criteria in Solid Tumors (RECIST 1.1). Tumor evaluation shall be performed every 6 weeks until disease progression, withdrawal from the study or death, initiation of new anti-tumor treatment or end of the study, whichever comes first. At the completion of C1, if the investigator judges that the subject cannot benefit from continuing to receive the study drug treatment, the subject shall withdraw from the trial.

**Determination of RD**

SRC shall comprehensively determine RD combined with MTD, PK data in the dose escalation stage and efficacy data observed in each dose group in the dose expansion research stage.

**SRC**

During the study, a Scientific Review Committee composed of coordinating investigators, principal investigators of enrolled centers, sponsor and/or CRO medical monitor, PK experts and biostatisticians shall be established to review safety data generated in the study, determine the allocation of subjects in each dose group and dose level in dose escalation research, and recommend the dose selected for expansion research and RD.

This study includes screening period, treatment period, safety follow-up period (28 days after the last administration) and survival follow-up period. During the survival follow-up period, the survival status of subjects and other anti-tumor treatment conditions shall be followed up.

All patients will receive study drug treatment until disease progression (based on RECIST 1.1 criteria), death, occurrence of intolerable toxicity or other conditions requiring termination of treatment.

**Research Subjects**

This study enrolls subjects with advanced malignant solid tumors (such as gastric cancer, esophageal cancer, urothelial carcinoma, prostate cancer, renal cancer, colorectal cancer, non-small cell lung cancer, triple-negative breast cancer, liver cancer, pancreatic cancer, head and neck squamous cell carcinoma, melanoma, cervical cancer, ovarian cancer, nasopharyngeal carcinoma, etc.).

**Inclusion and Exclusion Criteria**

**Inclusion Criteria**

Subjects must meet all the following criteria:
The subject must be informed of this study before the trial and voluntarily sign a written informed consent form;
Aged 18-80 years old (including boundary values), regardless of gender;
Patients with advanced solid tumors confirmed by histology and/or cytology, who have no standard treatment regimen,

failed standard treatment or are not suitable for standard treatment at this stage;
At least one evaluable tumor lesion in the dose escalation stage, and at least one measurable tumor lesion according to RECIST Version 1.1 in the dose expansion stage;
ECOG score 0~1;
Expected survival time ≥3 months;
Main organ functions are basically normal, and laboratory examination values during screening period meet the following standards:

**Table 8**

| System | Laboratory Examination Value |
|---|---|
| Hematology (No blood transfusion or hematopoietic stimulating factor treatment within 14 days) | |
| Absolute Neutrophil Count | ≥1.5 ×109/L |
| Platelet | ≥75×109/L |
| Hemoglobin | ≥90g/L |
| Renal Function | |
| Serum Creatinine | ≤1.5×ULN |
| Creatinine Clearance Rate (CrCl) (Calculated only when creatinine > 1.5× ULN) | ≥50 mL/min (Calculated according to Cockcroft-Gault formula) |
| Liver Function | |
| Total Bilirubin (TBIL) | ≤1.5 × ULN |
| Alanine Aminotransferase (ALT) | ≤3×ULN;<br>≤5×ULN for patients with liver metastasis or liver cancer |
| Aspartate Aminotransferase (AST) | ≤3×ULN;<br>≤5×ULN for patients with liver metastasis or liver cancer |
| Coagulation Function | |
| International Normalized Ratio (INR) or Activated Partial Thromboplastin Time (APTT) | ≤1.5 × ULN |
| Echocardiography | |
| Left Ventricular Ejection Fraction (LVEF) | ≥50% |
| Electrocardiogram | |
| Fridericia Corrected QT Interval (QTcF) | <450ms for male; <470ms for female |

Serum pregnancy test result of female subjects of childbearing age is negative;
The subject agrees to use reliable contraceptive methods from signing the informed consent form to 90 days after the last administration, including but not limited to abstinence, male vasectomy, female sterilization, effective intrauterine device, and effective contraceptive drugs.

**Exclusion Criteria**

Subjects meeting any of the following criteria shall be excluded from the trial:
Chemotherapy, radiotherapy, biological therapy, endocrine therapy, targeted therapy, immunotherapy and other anti-tumor therapies within 4 weeks before the first use of the study drug, except the following items:

Nitrosourea or Mitomycin C within 6 weeks before the first use of the study drug;

Oral fluorouracils and small molecule targeted drugs within 2 weeks before the first use of the study drug or 5 half-lives of the drug (whichever is longer);

Traditional Chinese medicine with anti-tumor indications within 2 weeks before the first use of the study drug;

Other unlisted clinical research drugs or treatment within 4 weeks before the first administration;

Previous immunotherapy and ≥Grade 3 irAE or ≥Grade 2 immune-related myocarditis;

Clinically uncontrolled third-space effusion judged by the investigator to be unsuitable for enrollment;

Acute coronary syndrome occurring within the previous 6 months, including myocardial infarction, unstable angina pectoris, symptomatic congestive heart failure (NYHA Class II-IV), aortic dissection, stroke or other Grade 3 and above cardiovascular and cerebrovascular events;

Severe cardiac rhythm or conduction abnormalities, such as ventricular arrhythmia requiring clinical intervention, Grade II-III atrioventricular block, etc.;

Any factors increasing the risk of QTc prolongation or arrhythmia, such as congenital long QT syndrome, family history of long QT syndrome, use of any concomitant drugs known to prolong QT interval;

Uncontrollable hypertension (systolic blood pressure ≥150mmHg or diastolic blood pressure ≥100mmHg after optimal medical treatment) or previous history of hypertensive emergency or hypertensive encephalopathy;

Symptomatic brain parenchymal metastasis or meningeal metastasis judged by the investigator to be unsuitable for enrollment;

Unable to swallow oral drugs, or having conditions seriously affecting gastrointestinal absorption judged by the investigator;

Human Immunodeficiency Virus (HIV) infected persons (HIV antibody positive);

Active hepatitis B (HBsAg positive and HBV-DNA>500IU/ml or the lower limit of detection of the research center [only applicable when the lower limit of detection of the research center is higher than 500IU/ml]), active hepatitis C (subjects with HCV antibody positive but HCV-RNA < the lower limit of detection of the research center can be enrolled), patients receiving preventive antiviral treatment other than interferon can be enrolled;

Active infection requiring intravenous anti-infection treatment at present;

Systemic glucocorticoids (prednisone>10mg/day or equivalent dose of similar drugs) or other immunosuppressive therapy within 14 days before the first administration, except the following situations:

Local, ocular, intra-articular, nasal and inhaled glucocorticoid treatment;

Short-term use of glucocorticoids for preventive treatment (such as preventing contrast agent allergy);

Live attenuated vaccine within 4 weeks before the first administration or planned to receive live attenuated vaccine during the study period;

Major organ surgery (excluding puncture biopsy) within 4 weeks before the first administration or significant trauma, or planned to receive elective surgery during the trial period;

Previous allogeneic hematopoietic stem cell transplantation or organ transplantation;

Known alcohol or drug dependence;

Mental disorders or poor compliance;

Adverse reactions of previous anti-tumor treatment not recovered to CTCAE Version 5.0 Grade ≤1 (except toxicities judged by the investigator without safety risks, such as alopecia, Grade 2 peripheral neurotoxicity, hypothyroidism stabilized by hormone replacement, etc.);

Strong CYP3A4 inhibitors or strong inducers within 7 days before the first administration (local medication is not restricted);

Pregnant or lactating patients;

The investigator considers that the subject has other reasons unsuitable for participating in this clinical study.

**Research Evaluation**

**Safety Evaluation**

Safety is evaluated through adverse events (graded by NCI-CTCAE Version 5.0), laboratory examinations, vital signs, physical examination, electrocardiogram, etc. Adverse events are recorded from the date the subject signed the informed consent form until 28 days after the last medication.

**Pharmacokinetic Evaluation**

PK blood samples are collected at scheduled time points to determine the plasma drug concentration of ILB-2109 tablets and estimate pharmacokinetic parameters. The pharmacokinetic characteristics of single and multiple administrations of ILB-2109 tablets monotherapy are evaluated.

**Pharmacodynamic Evaluation**

PD blood samples are collected at each PD blood collection point and baseline during the dose escalation stage of ILB-2109 tablets monotherapy in this study to determine the signal levels of pCREB, TNF-$\alpha$, IFN-$\gamma$ and IL-2 in blood cells for pharmacodynamic analysis, and the blood collection time is recorded in source documents and eCRF.

**Efficacy Evaluation**

Baseline confirmation and efficacy evaluation are performed by imaging examination (enhanced CT/plain CT, MRI, PET-CT). The same imaging evaluation method (including instrument parameters, operation standards, imaging planes, etc.) shall be used for the same subject during the trial period.

The evaluation standard adopts Response Evaluation Criteria in Solid Tumors (RECIST 1.1 Criteria).

Baseline evaluation is performed within 28 days before the first administration. Imaging evaluation is performed every 6$\pm$1 weeks after treatment initiation. For subjects still receiving treatment after 13 treatment cycles, imaging evaluation is performed every 12$\pm$4 weeks thereafter. Imaging examination time shall follow calendar days and shall not be adjusted due to treatment delay, and imaging examination shall be continued until disease progression, withdrawal from the study or death, initiation of new anti-tumor treatment or end of the study, whichever comes first.

**Study Endpoints**

**Primary Endpoints**

Incidence of dose-limiting toxicity (DLT) within 3 days of single administration and 21 days of continuous administration;

To determine the maximum tolerated dose (MTD) of ILB-2109 tablets and the appropriate recommended dose (RD) for subsequent studies.

**Secondary Endpoints**

**Safety Endpoints**

Incidence, type and toxicity grade of Treatment Emergent Adverse Event (TEAE) during treatment, graded according to National Cancer Institute Common Terminology Criteria for Adverse Events (NCI-CTCAE) Version 5.0;

Treatment Emergent Serious Adverse Events (TESAE) and toxic reactions leading to permanent drug discontinuation during treatment;

Safety laboratory examinations graded according to NCI-CTCAE (Version 5.0);

Vital signs, 12-lead electrocardiogram, physical examination and Eastern Cooperative Oncology Group (ECOG) score;

To evaluate PK parameters of ILB-2109 tablets, drug exposure (AUC0-last, AUC0-inf, AUC0-24), peak concentration (Cmax), peak time (Tmax), terminal elimination half-life (t1/2), apparent clearance rate (CL/F), apparent volume of distribution (V/F);

To preliminarily evaluate the effect of food on PK parameters of ILB-2109 tablets (expansion stage only), total drug exposure (AUC0-∞, AUC0-t), peak concentration (Cmax), peak time (Tmax), lag time (tlag), terminal elimination half-life (t1/2), apparent clearance rate (CL/F), apparent volume of distribution (V/F);

Safety and tolerability indicators in food effect research stage (expansion stage only): vital signs, 12-lead electrocardiogram, clinical laboratory examination indicators (blood routine, urine routine, blood biochemistry, etc.), physical examination, ECOG score, adverse events and serious adverse events.

**Efficacy Endpoints**

Objective Response Rate (ORR), Progression-Free Survival (PFS), Overall Survival (OS), 1-year OS rate, Time to Response (TTR), Duration of Response (DOR) and Clinical Benefit Rate (CBR).

**Exploratory Endpoints**

To explore the relationship between drug concentration of ILB-2109 tablets and signal levels of pCREB, TNF-α, IFN-γ and IL-2 in blood cells in monotherapy with ILB-2109 tablets;
To explore tumor biomarkers related to ILB-2109 tablets.

**Statistical Methods**

**Safety Analysis**

Safety analysis is based on Safety Analysis Set (SS). Descriptive statistics are used to analyze adverse events, laboratory examination indicators, vital signs, electrocardiogram, etc. The incidences of adverse events, serious adverse events and adverse events leading to drug discontinuation are summarized according to dose groups.
For the dose escalation stage, the DLT incidence of each dose group is calculated and presented with point estimate and two-sided 95% confidence interval by exact binomial method. The maximum tolerated dose (MTD) is determined according to the traditional "3+3" dose escalation design rule.

**Pharmacokinetic/Pharmacodynamic Analysis**

Pharmacokinetic analysis is based on PK Analysis Set (PKS). Plasma drug concentration of ILB-2109 tablets is determined, main PK parameters (Cmax, AUC, etc.) are estimated, drug clearance pathway is analyzed, and descriptive summary analysis is provided according to dose groups.
For all evaluable PD blood sample subjects, signal levels of pCREB, TNF-α, IFN-γ and IL-2 in blood cells at each PD blood collection point and baseline in the dose escalation stage are calculated, and descriptive statistical summary is performed.

**Efficacy Analysis**

Efficacy analysis is mainly based on Full Analysis Set (FAS). Per-protocol Set (PPS) is used as supplementary support if necessary. For efficacy data, Objective Response Rate (ORR) and Disease Control Rate (DCR) and their 95% confidence intervals are calculated. For Progression-Free Survival (PFS), Duration of Response (DOR) and Overall Survival (OS), Kaplan-Meier method is used for descriptive statistics, Greenwood method is used to provide 95% confidence intervals, and survival curve is drawn. Waterfall plot, spider plot and swimmer plot are drawn to present the

maximum change from baseline of the sum of target lesion long diameters of each patient, the change of tumor size over time and the occurrence of each clinical endpoint over time respectively.

**Recommended Dose (RD)**

RD is determined comprehensively according to safety evaluation, preliminary efficacy and PK data, combined with preclinical pharmacodynamic model data and in vitro research data.

**Table 9. Preliminary Efficacy Results of Subjects**

| Screeni ng No. | Tumor Type | Baseli ne | 6W | | 12W | | 18W | | 24W | | Tumor Evaluati on | Treatme nt Duration (d) | Previous Use of PD-1 |
|---|---|---|---|---|---|---|---|---|---|---|---|---|---|
| | | Target Lesio n mm | Target Lesio n mm | Evaluat ion Time (d) | Target Lesio n mm | Evaluat ion Time (d) | Target Lesio n mm | Evaluat ion Time (d) | Target Lesio n mm | Evaluat ion Time (d) | | | |
| 02004( 100mg ) | Rectal Can- cer | 131 | 136 | 52 | | | | | | | SD | 44 | Yes |
| 02005( 100mg ) | Lung Cancer | 43.9 | 43.1 | 57 | 47 | 85 | | | | | SD | 81 | No |
| 01002( 200mg ) | Cervical Cancer | 29.1 | 18.2 | 51 | 14.8 | 96 | 13.8 | 135 | | | PR | 161 | No |
| 01005( 200mg ) | Adenoid Cystic Carci- noma | 216.6 | 203.5 | 48 | 217.7 | 91 | 217.4 | 133 | 230.7 | 175 | SD | 43 | Yes |
| 01008( 300mg ) | Spindle Cell Sarcoma | 174.9 | 167.8 | 52 | 185.9 | 94 | 200.9 | 139 | | | SD | 173 | Yes |
| 03001( | Esophageal | | | | | | | | | | SD | 189 | Yes |
| 300mg ) | Cancer | | | | | | | | | | | | |
| 03003* (300m g) | Adenoid Cystic Carci- noma | 106.5 | 97.1 | 47 | 95.7 | 89 | | | | | SD | 147 | Yes |

Remarks: PD: Progressive Disease, the sum of the longest diameters of target lesions increases by at least ≥20%, or new lesions appear. SD: Stable Disease, the reduction of the sum of the longest diameters of target lesions does not reach PR, or the increase does not reach PD. PR: Partial Response, the sum of the longest diameters of target lesions decreases by ≥30%, maintained for at least 4 weeks. The esophageal cancer subject No. 03001 had no target lesions and was assessed as SD via non-target lesions, hence no numerical values.
Cut-off Time: July 20, 2023
*Still under treatment

**Table 10. Potential Efficacy Signals Observed Starting from 100mg Dose Group**

| Dose (mg) | Tumor Type | No. | Evaluation (6 Week) | Evaluation (12 Week) | Evaluation (18 Week) | Evaluation (24 Week) | Remarks on Previous PD-1 Use |
|---|---|---|---|---|---|---|---|
| 40 | Lung Adenocarci-no ma | 2001 | PD | - | - | - | Sintilimab |
| 40 | Colon Cancer | 2002 | PD | - | - | - | - |
| 40 | **Fallopian** Tube Cancer | 2003 | PD | - | - | - | Tislelizumab |
| 100 | Colorectal Cancer | 2004 | SD (↑) | - | - | - | Camrelizumab |
| 100 | Lung Adenocarci-no ma | 2005 | SD (↓5%) | PD | - | - | - |
| 100 | Lung Adenocarci-no ma | 2006 | PD | - | - | - | - |
| 200 | Prostate Cancer | 2007 | - | - | - | - | - |
| 200 | Esophageal Squamous Cell Carcinoma | 2008 | PD | - | - | - | Camrelizumab |
| 200 | Rectal Adenocar-cino ma | 1001 | - | - | - | - | - |
| 200 | Cervical Cancer | 1002 | PR | PR | PR(↓53%) | - | - |
| 200 | Lung Squamous Cell Carcinoma | 1004 | - | - | - | - | Sintilimab |
| 200 | Adenoid Cystic Carcinoma | 1005 | SD (↓6%) | SD (↑) | SD (↑) | SD (↑) | Sintilimab, Tori-palimab |
| 300 | Malignant Pleo-morphic Adeno-carcino ma | 1007 | - | - | - | - | Tislelizumab |
| 300 | Spindle Cell Sar-coma | 1008 | SD (↓4%) | SD (↑) | SD (↑) | - | Toripalimab |
| 300 | Esophageal Can-cer | 3001 | SD | SD | SD | SD | Camrelizumab |
| 300 | Rectal Adenocar-cino ma | 1009 | PD | - | - | - | Sintilimab |
| 300 | Rectal Cancer | 1010 | PD | - | - | - | - |
| 300 | Adenoid Cystic Carcinoma | 3003 | SD (↓8.9%) | SD (↓10.14%) | - | - | Tislelizumab |
| 400 | Colon Cancer | 1013 | - | - | - | - | Sintilimab |
| 400 | Ovarian Cancer | 1015 | PD | - | - | - | - |
| 400 | Lung Cancer | 1016 | - | - | - | - | Camrelizumab |
| 400 | Intrahepatic Cho-langiocarc inoma | 1017 | - | - | - | - | Pembrolizuma b |
| 100mg: 1 case of rectal cancer with SD, 1 case of lung cancer with SD<br>200mg: 1 case of cervical cancer with PR, 1 case of adenoid cystic carcinoma with SD<br>300mg: 1 case of spindle cell sarcoma with SD, 1 case of esophageal cancer with SD, 1 case of adenoid cystic carcinoma with SD | | | | | | | |

## Overview of Adverse Events Related to Test Drug

A total of 85 case-times of test drug-related Adverse Events (AE) occurred in 19 subjects. Common test drug-related AEs included vomiting, elevated bilirubin, proteinuria, elevated aspartate aminotransferase, anemia, elevated alanine aminotransferase, decreased appetite, etc., which were comparable to the incidence of AEs of similar products.

The above are only preferred specific embodiments of the present invention, but the protection scope of the present invention is not limited thereto. Any equivalent replacement or modification made by those skilled in the art within the technical scope disclosed by the present specification according to the technical solution and inventive concept of the present invention shall fall within the protection scope of the present invention.

## Claims

1. Use of the following compound or a pharmaceutically acceptable salt thereof in the preparation of a medicament, wherein the medicament is used for treating cancer:

   wherein:

   $R_1$ is ($C_1$-$C_6$) alkyl optionally substituted with hydroxyl;
   $R_2$ is independently halogen, ($C_1$-$C_6$) alkyl or halogen-substituted ($C_1$-$C_6$) alkyl;
   $R_3$ is independently ($C_1$-$C_6$) alkyl;
   m is 0, 1, 2, 3 or 4;
   n is 0, 1, 2, 3, 4 or 5.

2. The use according to Claim 1, wherein $R_1$ is hydroxyl-substituted ($C_1$-$C_6$) alkyl, preferably hydroxyl-substituted ($C_1$-$C_3$) alkyl, more preferably hydroxymethyl.

3. The use according to Claim 1, wherein $R_2$ is independently trifluoromethyl or ($C_1$-$C_6$) alkyl.

4. The use according to Claim 1, wherein the compound is

NH2
N
N
N
HO
N
F3C
N

5. The use according to Claim 1, wherein the cancer is a solid tumor.

6. The use according to Claim 1, wherein the cancer is selected from gastric cancer, esophageal cancer, urothelial carcinoma, prostate cancer, renal cancer, colorectal cancer, non-small cell lung cancer, triple-negative breast cancer, liver cancer, pancreatic cancer, head and neck squamous cell carcinoma, melanoma, cervical cancer, ovarian cancer, endometrial cancer, sarcoma, glioma, rectal cancer, lung cancer, adenoid cystic carcinoma, spindle cell sarcoma, lung adenocarcinoma, colon cancer, fallopian tube cancer, esophageal squamous cell carcinoma, rectal adenocar-

cinoma, lung squamous cell carcinoma, malignant pleomorphic adenocarcinoma, intrahepatic cholangiocarcinoma or nasopharyngeal carcinoma.

7. The use according to Claim 4, wherein the compound has Crystal Form A, whose X-ray powder diffraction pattern has characteristic diffraction peaks at the following 2θ angles: 8.08±0.2°, 11.30±0.2°, 14.00±0.2°, 16.90±0.2°, 18.30 ±0.2°, 22.52±0.2°, 23.15±0.2°, 25.26±0.2°.

8. The use according to Claim 4, wherein the compound has Crystal Form A, and its X-ray powder diffraction pattern is as shown in FIG. 4.

9. The use according to Claim 1, wherein the compound is a deuterated compound, and preferably, the deuterated compound is selected from the group consisting of:

.

**10.** The use according to Claim 1, wherein the medicament further comprises an anti-CTLA-4 monoclonal antibody, preferably, the weight ratio of the compound to the anti-CTLA-4 monoclonal antibody is 5:1 to 50:1, such as 10:1 to 20:1, and preferably, the medicament is used for treating colon cancer.

3001 esophageal cancer
1005 adenoid cystic carcinoma
1008 spindle cell sarcoma
1002 cervical cancer
3003 adenoid cystic carcinoma
2005 lung adenocarcinoma
2006 lung adenocarcinoma
2004 rectal adenocarcinoma
2002 colon cancer
1001 rectal adenocarcinoma
1009 rectal adenocarcinoma
1013 colon cancer
1010 rectal cancer
2001 lung adenocarcinoma
1017 intrahepatic cholangiocarcinoma
1015 ovarian cancer
2003 fallopian tube cancer
1004 lung squamous cell carcinoma
1007 malignant pleomorphic adenocarcinoma
2007 prostate cancer
01016 lung cancer
2008 esophageal squamous cell carcinoma
subject
PR
SD
PD
+ D/C due to PD
☆D/C due to AE or other reasons
Dose Group
40mg
100mg
200mg
300mg
400mg
0
20
40
60
80
100
120
140
160
180
200
Treatment Duration
Cut-off Date: July 20，2023

Figure 1

Adverse Events Related to the Test Drug
vomiting
13
elevated bilirubin
8
proteinuria
7
elevated aspartate aminotransferase
4
anemia
4
elevated alanine aminotransferase
4
decreased appetite
3
fatigue
3
elevated white blood cell count
3
elevated gamma-glutamyl transferase
3
elevated alkaline phosphatase
2
hyperglycemia
2
nausea
2
hyponatremia
2
hypoproteinemia
2
0
2
4
6
8
10
12
14

Figure 2

Occurrence of Adverse Events Related to the Test Drug in Each Dose Group
35
30
25
20
15
10
5
0
11
10
21
33
10
40mg(N=3)
100mg(N=3)
200mg(N=6)
300mg(N=6)
400mg(N=3)

Figure 3

Intensity (Counts)
7000
6000
5000
4000
3000
2000
1000
0
5
10
15
20
25
30
35
2θ angles (°)

Figure 4

## INTERNATIONAL SEARCH REPORT

International application No.
**PCT/CN2024/131172**

**A. CLASSIFICATION OF SUBJECT MATTER**

C07D487/04(2006.01)i；A61K31/505(2006.01)i；A61K31/519(2006.01)i；A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, WPABS, WPABSC, DWPI, ENTXT, ENTXTC, CJFD, MEDNPL, VEN, VCN, STN, PUBMED, ISI-WEB OF SCIENCE, CNKI, 百度, BAIDU, Bing: 英诺湖医药, 夏明德, 陈如雷, 张倩, 李燕, 三唑, 嘧啶, 癌症, 肿瘤, 实体瘤, 结肠癌, 胃癌, 食管癌, 前列腺癌, 肾癌, 结直肠癌, 非小细胞肺癌, 乳腺癌, 肝癌, 胰腺癌, 黑色素瘤, 宫颈癌, 卵巢癌, 子宫内膜癌, 肉瘤, 胶质瘤, 直肠癌, 肺癌, 鼻咽癌, 氘代, CTLA-4, CTLA4, 结构检索, structural search, Innolake Biopharm, cancer, tumor, solid tumor, colon cancer, gastric cancer, esophageal cancer, prostatic cancer, kidney cancer, colorectal cancer, non-small cell lung cancer, breast cancer, liver cancer, pancreatic cancer, melanoma, cervical cancer, ovarian cancer, endometrial cancer, sarcoma, glioma, rectal cancer, lung cancer, nasopharyngeal carcinoma, Deuterated, Deuterium

**C. DOCUMENTS CONSIDERED TO BE RELEVANT**

| Category* | Citation of document, with indication, where appropriate, of the relevant passages | Relevant to claim No. |
|---|---|---|
| X | WO 2019206336 A1 (MEDSHINE DISCOVERY INC.) 31 October 2019 (2019-10-31) claims 1-3 and 8, description, page 2, last paragraph to page 3, paragraph 1, figure 1, and description, page 1, paragraph 3 | 1-8, 10 |
| Y | WO 2019206336 A1 (MEDSHINE DISCOVERY INC.) 31 October 2019 (2019-10-31) claims 1-3 and 8, description, page 2, last paragraph to page 3, paragraph 1, figure 1, and description, page 1, paragraph 3 | 1-3, 5-6, 9 |
| X | WO 2018184590 A1 (MEDSHINE DISCOVERY INC.) 11 October 2018 (2018-10-11) claims 1-21, and description, page 1, paragraph 4, page 29, paragraph 4, and page 31, paragraph 3 | 1-6, 10 |
| Y | WO 2018184590 A1 (MEDSHINE DISCOVERY INC.) 11 October 2018 (2018-10-11) claims 1-21, and description, page 1, paragraph 4, page 29, paragraph 4, and page 31, paragraph 3 | 1-3, 5-6, 9 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 January 2025** | **26 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/131172**

**C. DOCUMENTS CONSIDERED TO BE RELEVANT**

| Category* | Citation of document, with indication, where appropriate, of the relevant passages | Relevant to claim No. |
|---|---|---|
| Y | CN 109535161 A (JIANGSU HENGRUI PHARMACEUTICALS CO., LTD. et al.) 29 March 2019 (2019-03-29) claims 1-3 and 17-18, and description, paragraph [0144] | 1-3, 5-6, 9 |
| A | CN 111601809 A (DIZAL (JIANGSU) PHARMACEUTICAL CO., LTD.) 28 August 2020 (2020-08-28) claims 1-40 | 1-10 |
| A | CN 114585625 A (INCYTE CORP.) 03 June 2022 (2022-06-03) claims 1-15, and description, paragraphs [0013] and [0303] | 1-10 |
| A | CN 112384515 A (INCYTE CORP.) 19 February 2021 (2021-02-19) claims 1-50, and description, paragraphs [0934]-[0938] | 1-10 |

**INTERNATIONAL SEARCH REPORT**
**Information on patent family members**

International application No.
**PCT/CN2024/131172**

| Patent document cited in search report | Publication date (day/month/year) | Patent family member(s) | Publication date (day/month/year) |
|---|---|---|---|
| WO 2019206336 A1 | 31 October 2019 | PT 3766884 T | 31 March 2022 |
| | | US 2021094957 A1 | 01 April 2021 |
| | | US 11649239 B2 | 16 May 2023 |
| | | ES 2908424 T3 | 29 April 2022 |
| | | EP 3766884 A1 | 20 January 2021 |
| | | EP 3766884 A4 | 31 March 2021 |
| | | EP 3766884 B1 | 09 February 2022 |
| | | DK 3766884 T3 | 28 March 2022 |
| | | PL 3766884 T3 | 18 July 2022 |
| | | JP 2021522335 A | 30 August 2021 |
| | | JP 7406691 B2 | 28 December 2023 |
| WO 2018184590 A1 | 11 October 2018 | DK 3611174 T3 | 04 July 2022 |
| | | PL 3611174 T3 | 08 August 2022 |
| | | JP 2020516606 A | 11 June 2020 |
| | | JP 7065113 B2 | 11 May 2022 |
| | | US 2020131184 A1 | 30 April 2020 |
| | | US 11117899 B2 | 14 September 2021 |
| | | EP 3611174 A1 | 19 February 2020 |
| | | EP 3611174 A4 | 09 September 2020 |
| | | EP 3611174 B1 | 08 June 2022 |
| | | ES 2919474 T3 | 26 July 2022 |
| | | PT 3611174 T | 07 July 2022 |
| CN 109535161 A | 29 March 2019 | None | |
| CN 111601809 A | 28 August 2020 | BR 112021004774 A2 | 03 August 2021 |
| | | EP 3849983 A1 | 21 July 2021 |
| | | EP 3849983 A4 | 01 June 2022 |
| | | CA 3111869 A1 | 19 March 2020 |
| | | JP 2024123079 A | 10 September 2024 |
| | | US 2021009600 A1 | 14 January 2021 |
| | | US 11629147 B2 | 18 April 2023 |
| | | JP 2022500402 A | 04 January 2022 |
| | | JP 7572353 B2 | 23 October 2024 |
| | | AU 2019340767 A1 | 08 April 2021 |
| | | AU 2019340767 B2 | 14 November 2024 |
| | | AU 2019340767 B9 | 05 December 2024 |
| | | MX 2021002998 A | 14 May 2021 |
| | | AR 116315 A1 | 21 April 2021 |
| | | TW 202024089 A | 01 July 2020 |
| | | TWI 820209 B | 01 November 2023 |
| | | WO 2020052631 A1 | 19 March 2020 |
| | | KR 20210075996 A | 23 June 2021 |
| | | US 2020331918 A1 | 22 October 2020 |
| | | US 10858365 B2 | 08 December 2020 |
| CN 114585625 A | 03 June 2022 | AU 2020337350 A1 | 10 March 2022 |
| | | ECSP 22022322 A | 31 May 2022 |
| | | US 2021061809 A1 | 04 March 2021 |
| | | MX 2022002219 A | 14 June 2022 |
| | | PE 20230372 A1 | 06 March 2023 |
| | | JP 2022545923 A | 01 November 2022 |
| | | IL 290529 A | 01 April 2022 |

Form PCT/ISA/210 (patent family annex) (July 2022)

**INTERNATIONAL SEARCH REPORT**
**Information on patent family members**

International application No.
**PCT/CN2024/131172**

| Patent document cited in search report | Publication date (day/month/year) | Patent family member(s) | Publication date (day/month/year) |
|---|---|---|---|
| | | TW 202115082 A | 16 April 2021 |
| | | KR 20220066074 A | 23 May 2022 |
| | | US 2023124485 A1 | 20 April 2023 |
| | | CL 2022000457 A1 | 21 October 2022 |
| | | CO 2022003457 A2 | 20 May 2022 |
| | | AR 119822 A1 | 12 January 2022 |
| | | BR 112022003408 A2 | 17 May 2022 |
| | | EP 4021907 A1 | 06 July 2022 |
| | | WO 2021041360 A1 | 04 March 2021 |
| | | CA 3150766 A1 | 04 March 2021 |
| | | CR 20220124 A | 15 June 2022 |
| CN 112384515 A | 19 February 2021 | CR 20200441 A | 15 March 2021 |
| | | TW 202000666 A | 01 January 2020 |
| | | TWI 853802 B | 01 September 2024 |
| | | CR 20230030 A | 10 March 2023 |
| | | WO 2019168847 A1 | 06 September 2019 |
| | | BR 112020017421 A2 | 22 December 2020 |
| | | IL 303087 A | 01 July 2023 |
| | | IL 303087 B1 | 01 August 2024 |
| | | IL 303087 B2 | 01 December 2024 |
| | | KR 20200139153 A | 11 December 2020 |
| | | IL 276873 A | 29 October 2020 |
| | | EP 3759112 A1 | 06 January 2021 |
| | | ECSP 20060827 A | 31 December 2020 |
| | | US 2023357255 A1 | 09 November 2023 |
| | | CL 2020002198 A1 | 29 January 2021 |
| | | JP 2023123569 A | 05 September 2023 |
| | | PH 12020551332 A1 | 06 September 2021 |
| | | US 2019292188 A1 | 26 September 2019 |
| | | TW 202400599 A | 01 January 2024 |
| | | AU 2022283611 A1 | 02 February 2023 |
| | | CO 2020011908 A2 | 30 October 2020 |
| | | MX 2022014648 A | 15 December 2022 |
| | | US 2021139485 A1 | 13 May 2021 |
| | | US 11673894 B2 | 13 June 2023 |
| | | JP 2021515036 A | 17 June 2021 |
| | | JP 7474709 B2 | 25 April 2024 |
| | | CL 2022003185 A1 | 19 May 2023 |
| | | PE 20211001 A1 | 01 June 2021 |
| | | MX 2020008949 A | 08 January 2021 |
| | | CA 3092470 A1 | 06 September 2019 |
| | | MA 52422 A | 06 January 2021 |
| | | AU 2019227607 A1 | 15 October 2020 |
| | | AU 2019227607 B2 | 27 April 2023 |
| | | AU 2019227607 C1 | 20 July 2023 |
| | | BR 122023024273 A2 | 20 February 2024 |

Form PCT/ISA/210 (patent family annex) (July 2022)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description

- CN 110446712 B
- CN 112105617 B